# EUROPEAN PATENT APPLICATION

(11) **EP 2 497 530 A2**
(43) Date of publication of application: **12.09.2012**
(21) Application number: 12158427.0
(22) Date of filing: 07.03.2012
(51) Int. Cl.: A61N 1/36, A61N 1/378, A61N 1/372

(54) **Modular nerve stimulation system**

(30) Priority: 07.03.2011 US 201161450030 P
(71) Applicant: Barolat, Giancarlo, Golden, CO 80401 (US); Drees, Scott F., Dallas, TX 75219 (US); Kaula, Norbert F., Arvada, CO 80005 (US)
(72) Inventor: Barolat, Giancarlo, Golden, CO 80401 (US); Drees, Scott F., Dallas, TX 75219 (US); Kaula, Norbert F., Arvada, CO 80005 (US)
(74) Representative: Lecomte & Partners

(57) **Abstract**

The present disclosure provides a modular neurostimulator system. The system includes a stage one implant that can be externally powered and controlled. The stage one implant is made of one or more leads as well as a passive receiver and an external controlling and powering device. The receiver and the external device can be utilized either as a long-term trial system, or as a permanent system (depending on the patient's wishes, reimbursement, payor policy, etc). Since the receiver and the external device can have limited costs and features, they are a good vehicle for a long-term trial without risk of infection and excessive upfront cost. The length of the trial assures extreme accuracy in the evaluation of the efficacy of the modality. The system may also include a stage two implant that includes an implantable power supply and/or control elements connectable to one or more previously implanted stage one implants.

## Description

### BACKGROUND

As medical device technologies continue to evolve, neurostimulator devices have gained much popularity in the medical field. Neurostimulator devices are typically battery-powered devices that are designed to deliver electrical stimulation to a patient. Through proper electrical stimulation, the neurostimulator devices can provide pain relief for patients. In effect, the electrical signals sent by the neurostimulator devices "mask" or modify the pain signals before the pain signals reach the patient's brain. As a result, the patient may feel only a tingling sensation instead of pain in the area that is stimulated.

A typical implantable neurostimulator device may include one or more integrated circuit chips containing the control circuitry and neurostimulation circuitry. The neurostimulator device may also include a plurality of electrodes that are in contact with different areas of a patient's body. The implantable neurostimulator typically includes a battery, either permanent or rechargeable, that is utilized to power the stimulation circuitry and the external communications. Controlled by the control circuitry within the neurostimulator, the electrodes are each capable of delivering electrical stimulation to their respective target contact areas. Thus, the patient can use the neurostimulator device to stimulate areas in a localized manner.

Unfortunately, not all patients receive relief from the electrical stimulation provided by the neurostimulator. In some situations, physicians attempt to trial the electrodes placement by implanting them in the desired location and running the leads through the skin. Such trialing can only be utilized for a short period of time as there is a risk of infection through the open wound. The relatively short trial period greatly increases the chances that the patient might not be able to thoroughly assess the efficacy or lack thereof of the stimulation. This could lead to permanent implantation of a system in patients who did not have adequate pain relief, or explant in patients who might otherwise have benefitted from the stimulation, given a more appropriate trial length.. In addition, implantable neurostimulators are complex devices that are very expensive to purchase and implant. Given the uncertainty in the outcome and the high cost of the procedures, patients, physicians and third party payors are less likely to pursue treatment with neurostimulators and many patients do not have access to the beneficial therapeutic effects of neurostimulation.

Peripheral nerve stimulation has been used to treat chronic pain emanating from a patient's extremity, such as in the patient's arm and/or leg. However, a recurring difficulty associated with the treatment of pain within an extremity is the limited placement options available because of the often difficult necessity to place elements of the implantable neurostimulation system on two sides of a joint. This has been due to the size of the IPG (which include the lithium battery) which, in most individuals, makes it unsuitable for placement in a limb. By way of example, an electrode implantation on the tibial nerve in the ankle will require the implantable pulse generator (IPG) to be placed in the thigh, so that the implanted wires will have to cross the knee joint. However, as can be appreciated, repeated motion of the knee can easily cause structural damage to the lead, migration of the electrode, failed electrical connections and the like, thereby necessitating the repair of the lead, replacement of the electrode, or even removal of the neurostimulation system. At a minimum, such problems often result in further pain to the patient.

Therefore, while existing neurostimulator devices and techniques for their placement have been generally adequate for their intended purposes, they have not been entirely satisfactory in every aspect.

### SUMMARY

One of the broader forms of the present disclosure involves an electrical stimulation apparatus. In one aspect the stimulation system and techniques for implantation provide a bridge to implantation of a more permanent implantable pulse generator. In one embodiment, there is a first stimulation stage utilizing a reduced component stimulation device implantable in the patient. This device utilizes external controls and an external power source to function. External components are usable with the stage one device to control and power the stage one implantable components to provide stimulation. If stage one proves to be successful, one or more of the implanted components of stage one remain in the patient and are connected with an implantable pulse generator. In one form, the stage one device includes a passive receiver that is releasably coupled to a lead with stimulation electrodes. In this form, the lead may be disconnected from the passive receiver of stage one and replaced with an implantable pulse generator to form a stage two stimulation system. In an alternative form, the passive receiver includes one or more expansion connections and one or more additional components are added to the expandable passive receiver of stage one to form a modular implantable pulse generator in stage two.

In one specific embodiment, a method of limb peripheral nerve stimulation for a patient is provided that includes positioning a stimulation electrode adjacent the peripheral nerve, the electrode extending from a lead, the lead releasably coupled to a passive receiver, such that it does not extend across a movable joint. As an additional feature, if stimulation of the electrode provides pain relief to the patient, an additional aspect of the method can include coupling an implantable pulse generator to the lead.

In a further embodiment, a method of peripheral nerve stimulation includes a staged implantation procedure. The first stage includes positioning a stimulation electrode adjacent the peripheral nerve, the electrode extending from a lead coupled to a passive receiver, the passive receiver having a first displacement volume. The passive receiver is implanted in a pocket in the patient's tissue and the pocket closes around the passive receiver. The passive receiver is controlled to the stimulate the patient via an external pulse generator. If the first stage proves successful, a second stage implantation procedure is performed to implant an implantable pulse generator component. The second stage includes accessing the pocket having the passive receiver and coupling an implantable pulse generator to the lead, the implantable pulse generator substantially matching the first displacement volume occupied by the passive receiver and having a power source and a controller to control pulse delivered to the stimulation electrode. In one aspect, the passive receiver is removed from the pocket before the implantable pulse generator is implanted. In another aspect, a portion of the passive receiver is removed from the pocket and one or more modules are added to the passive receiver to form an implantable pulse generator.

In another form, a modular stimulation system for nerve stimulation is provided that includes a plurality of stimulation electrodes connected to a lead having a distal portion and an opposing proximal portion forming a first portion of a detachable coupling assembly. The modular system includes a passive receiver having a distal portion forming a second portion of a detachable coupling assembly complimentary to the first portion to electrically couple the passive receiver to the lead, the passive receiver configured to convert wireless energy to a pulse suitable to energize the plurality of stimulation electrodes. The system also includes an implantable pulse generator having an internal power source and a controller to control delivery of electrical stimulation pulses, the implantable pulse generator having a distal portion forming a third portion of a detachable coupling assembly complimentary with the first portion to electrically couple the implantable pulse generator to the lead. The passive receiver and the implantable pulse generator are configured to be interchangeably coupled to the first portion of the detachable coupling.

In still a further form, a modular stimulation system for nerves is provided that can be modified after implantation. The system includes a plurality of stimulation electrodes with a lead having a distal portion electrically coupled to the electrodes and an opposing proximal portion forming a first portion of a detachable coupling assembly. A first portion of the system includes a passive receiver having an expansion connection and a distal portion forming a second portion of a detachable coupling assembly complimentary to the first portion to electrically couple the passive receiver to said lead. The passive receiver being configured to convert wireless energy to a pulse suitable to energize the plurality of stimulation electrodes. The modular system also includes an implantable pulse generator having at least an internal power source or a controller to control delivery of electrical stimulation pulses, the implantable pulse generator having a portion forming an expansion coupling assembly complimentary with the expansion connection to electrically couple the implantable pulse generator to the passive receiver.

In yet a further aspect, a method of treating pain is also provided. The method includes implanting a stimulation system adjacent a nerve to relieve nociceptive pain prior to a surgical intervention to correct the injury causing the nociceptive pain, stimulating the nerve to relieve nociceptive pain, and operating on a source of the nociceptive pain while the stimulation system remains implanted in the patient.

In still a further aspect, there is a provided a method of treating phantom limb pain. The method includes implanting a stimulation system adjacent a nerve associated with the intact limb to be amputated and stimulating the nerve with the limb remaining intact. The method continues with amputating the limb while at least a portion of the stimulation system remains implanted in the patient and operating the stimulation system to stimulate at least one nerve associated with the amputated limb.

As a further feature, the present disclosure provides a modular neurostimulation system that can be implanted in a patient in stages. The first neurostimulation stage component is configured to provide electrical stimulation from an implanted device that is entirely implanted under the skin to inhibit the risk of infection. In one aspect, the method includes designing the implantable first stage components to have simplified electrical components that can be manufactured at a relatively low first cost. The implantable first stage can then be offered to a customer at a correspondingly low first sales price. The implantable first stage relies on external components to power and/or control the stimulation signals generated by the first stage. The contemplated staged implant procedure includes designing a second implantable stage component having substantially more complex components sufficient to form a self contained implantable pulse generator. The second stage components being configured to operative engage one or more components of the first stage device previously implanted. The implantable pulse generator being manufactured at a cost substantially greater than the first stage device and being offered to the customer at a sales price that is more than double the sales price of the first stage component. As a result, the low cost first stage may be utilized by the patient to determine efficacy and the much more expensive second stage only being purchased after the first stage proves successful.

Various embodiments of the present inventions are set forth in the attached figures and in the Detailed Description as provided herein and as embodied by the claims. It should be understood, however, that this Summary does not contain all of the aspects and embodiments of the one or more present inventions, is not meant to be limiting or restrictive in any manner, and that the invention(s) as disclosed herein is/are understood by those of ordinary skill in the art to encompass obvious improvements and modifications thereto.

Additional advantages of the present invention will become readily apparent from the following discussion, particularly when taken together with the accompanying drawings.
Further, the present invention is also directed to the features according to the following sections. The features of these sections may also be combined with any other features of the present specification.
1. A method of limb peripheral nerve stimulation for a patient, comprising:
   gaining access to a peripheral nerve;
   positioning a stimulation electrode adjacent the peripheral nerve, the electrode extending from a lead, the lead releasably coupled to a passive receiver stimulator;
   implanting the passive receiver and lead into the patient;
   providing an external pulse generator capable of communicating a pulse to the passive receiver through the patient's skin; and
   energizing the external pulse generator thereby actuating the passive receiver to energize the stimulation electrode positioned adjacent the peripheral nerve
   evaluating the efficacy of stimulation during a first nerve stimulation stage utilizing the passive receiver stimulator;
   accessing the lead; and
   coupling an implantable pulse generator to the lead, the implantable pulse generator including a power source and control system to independently energize the stimulation electrode positioned adjacent the peripheral nerve during a second nerve stimulation stage.
2. The method according to section 1, wherein said providing includes securing the external pulse generator adjacent the passive receiver.
3. The method according to section 1 or 2, wherein said securing includes placing a band around a limb of the patient.
4. The method according to any one of the preceding sections, wherein said implanting occurs on an extremity of a patient.
5. The method according to any of one of the preceding sections, further including decoupling the passive receiver from the lead prior to coupling the implantable pulse generator.
6. The method according to any one of the preceding sections, wherein said accessing includes forming an opening in the patient's skin and further including closing the opening after said coupling.
7. The method according to any one of the preceding sections, further including electrically coupling an implantable pulse generator directly to said lead.
8. The method according to any one of the preceding sections, wherein said coupling includes electrically coupling the implantable pulse generator to the passive receiver.
9. The method according to any one of the preceding sections, wherein said implanting includes forming a pocket within the tissue of the patient sized to receive the passive receiver, and after said implanting, closing the pocket with the passive receiver implanted therein; and further including after said energizing, the steps of accessing the pocket; disconnecting the lead from the passive receiver; removing the passive receiver from the patient; connecting an implantable pulse generator to the lead; positioning the implantable pulse generator in the same pocket; and closing the pocket.
10. The method according to any one of the preceding sections, wherein the passive receiver has a first displacement volume and the implantable pulse generator has a second displacement volume, wherein the second displacement volume substantially matches the first displacement volume so as to fit in the pocket formed to receive the passive receiver.
11. The method according to any one of the preceding sections, further including accessing the passive receiver and coupling at least a power unit to the passive receiver to supply stimulation power to the passive receiver, and closing the access passage through the patient's skin.
12. A method of peripheral nerve stimulation for a patient, comprising:
   gaining access to a peripheral nerve;
   positioning a stimulation electrode adjacent the peripheral nerve, the electrode extending from a lead coupled to a passive receiver, the passive receiver having a first length, a first height and a first width defining a first displacement volume;
   forming a pocket in the patient's tissue;
   implanting the passive receiver in the pocket;
   closing the pocket;
   providing an external pulse generator capable of communicating a pulse to the passive receiver through the patient's skin to thereby energize the stimulation electrode in a first stage of nerve stimulation;
   determining efficacy of the stimulation electrode;
   accessing the pocket;
   coupling an implantable pulse generator to the lead, the implantable pulse generator including a power source and a controller to control pulse delivered to the stimulation electrode during a second stage of nerve stimulation;
   implanting the implantable pulse generator in the pocket within substantially the first displacement volume occupied by the passive receiver; and
   closing the pocket.
13. The method according to section 12, wherein the implantable pulse generator has a second displacement volume substantially matching said first displacement volume of the passive receiver.
14. The method according to section 13, wherein the implantable pulse generator has dimensions substantially matching the first height, first length and first depth of the passive receiver.
15. The method according to any one of sections 12-14, further including decoupling the passive receiver from the lead prior to coupling the implantable pulse generator.
16. The method according to any one of sections 12-15, wherein said passive receiver includes at least one removable component covering a connection and said coupling an implantable pulse generator includes connecting at least a power supply to the connection of the passive receiver to form a modular implantable pulse generator.
17. The method according to any one of sections 12-16, wherein said passive receiver includes at least on removable component, and further including removing the removable component prior to said coupling the implantable pulse generator.
18. The method according to section 17, wherein the removable component has a component displacement volume and the implantable pulse generator has a second displacement volume, wherein the component displacement volume substantially matches the second displacement volume.
19. A method of treating pain, comprising
   implanting a stimulation system adjacent a nerve to relieve nociceptive pain prior to a surgical intervention to correct the injury causing the nociceptive pain;
   stimulating the nerve to relieve nociceptive pain; and
   operating on a source of the nociceptive pain while the stimulation system remains implanted in the patient.
20. The method according to section 19, further including stimulating the nerve after said operating.
21. The method according to section 19 or 20, wherein the nociceptive pain is in a joint and said stimulating occurs for at least a week before said operating.
22. The method according to section 21, wherein the nociceptive pain is in the knee and said implanting including positioning an electrode adjacent the femoral nerve and implanting a stimulator between the knee and the hip joints.
23. A method of treating phantom limb pain, comprising:
   implanting a stimulation system adjacent a nerve associated with the intact limb to be amputated;
   stimulating the nerve to with the limb remaining intact;
   amputating the limb while the stimulation system remains implanted in the patient; and
   operating the stimulation system to stimulate the nerve associated with the amputated limb.
24. The method according to section 23, wherein said stimulating occurs for at least a week before said amputating.
25. A method of providing a staged neurostimulation system, comprising:
   providing an implantable first stage assembly having an electrode array with a lead defining a coupling assembly and a passive receiver configured for mating with the coupling assembly;
   offering the passive receiver for a first sales price;
   providing an implantable second stage assembly having an implantable pulse generator configured for mating with the lead coupling assembly, wherein the implantable pulse generator is configured to be exchanged for the passive receiver and may be interchangeably connected to the lead coupling assembly; and
   offering the implantable second stage for a second sales price that is at least twice as large as the first sales price.
26. The method according to section 25, wherein the second sales prices is at least three times greater than the first sales price.
27. The method according to section 25, wherein the second sales prices is at least five times greater than the first sale price.
28. The method according to any one of sections 25-27, wherein the passive receiver has a first displacement volume and the implantable pulse generator has a second displacement volume substantially matching the first displacement volume.

### BRIEF DESCRIPTION OF THE DRAWINGS

Aspects of the present disclosure are best understood from the following detailed description when read with the accompanying figures. It is emphasized that, in accordance with the standard practice in the industry, various features are not drawn to scale. In fact, the dimensions of the various features may be arbitrarily increased or reduced for clarity of discussion.

Fig. 1 is stylized overview of the human nervous system.

Fig. 2 is a simplified block diagram of a first configuration of a neurostimulator utilizing an external power supply.

Fig. 3 is a simplified block diagram of a second configuration of the neurostimulator utilizing an implanted power supply with associated stimulation control circuitry.

Fig. 4a is a diagrammatic block diagram of a further embodiment of an expandable neurostimulator utilizing an external power supply.

Fig. 4b is a diagrammatic block diagram of the neurostimulator of Fig. 4a modified to include an implanted power supply and associated stimulation control circuitry.

Fig. 5a is a diagrammatic top view block diagram of a further neurostimulator according to another aspect of the present invention.

Fig. 5b is a side view of the neurostimulator of Fig. 5a.

Figs. 6a-c illustrate a further embodiment of a modular neurostimulation system.

Fig. 7a is a top view of a further neurostimulator.

Fig. 7b is a side view of the neurostimulator of Fig. 6a.

Fig. 8 is flow diagram of a staged neurostimulation method.

Fig. 9 is a flow diagram of an initial stage of a staged neurostimulation method.

Fig. 10 is a flow diagram of a second stage of a staged neurostimulation method.

Figs. 11a-c illustrate a staged implantation technique for a modular neurostimulation system according to one aspect of the present invention.

Fig. 12 illustrates a stylized view of a portion of the human body with neurostimulators implanted according to further aspects of the present invention.

Fig. 13 is an external pulse generator in combination with a retention sleeve.

### DETAILED DESCRIPTION

It is to be understood that the following disclosure provides many different embodiments, or examples, for implementing different features of the invention. Specific examples of components and arrangements are described below to simplify the present disclosure. These are, of course, merely examples and are not intended to be limiting. Various features may be arbitrarily drawn in different scales for simplicity and clarity.

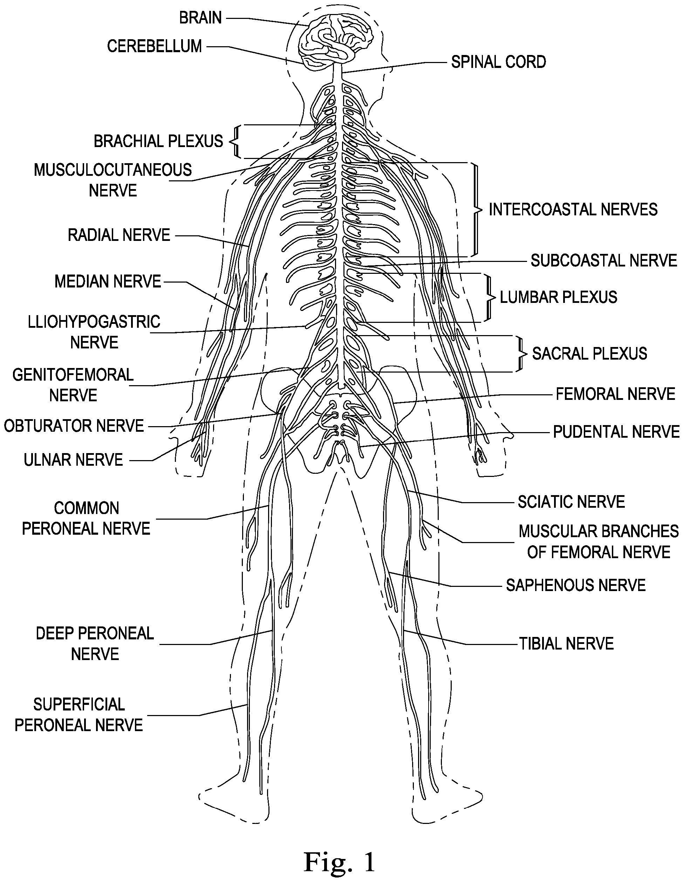

The human nervous system includes a complex network of neurological structures that extend throughout the body. As shown in Fig. 1, the brain interconnects with the spinal cord which branches into the brachial plexus near the shoulders and the lumbar plexus and sacral plexus in the lower back. The limb peripheral nerves of the arms extend distally from the brachial plexus down each arm. Similarly, the limb peripheral nerves of the legs extend distally from the lumbar plexus and sacral plexus. A number of the larger limb peripheral nerves are identified in Fig. 1. As discussed further below, certain aspects of the present invention are particularly well suited to stimulation of limb peripheral nerves, including those identified in Fig. 1.

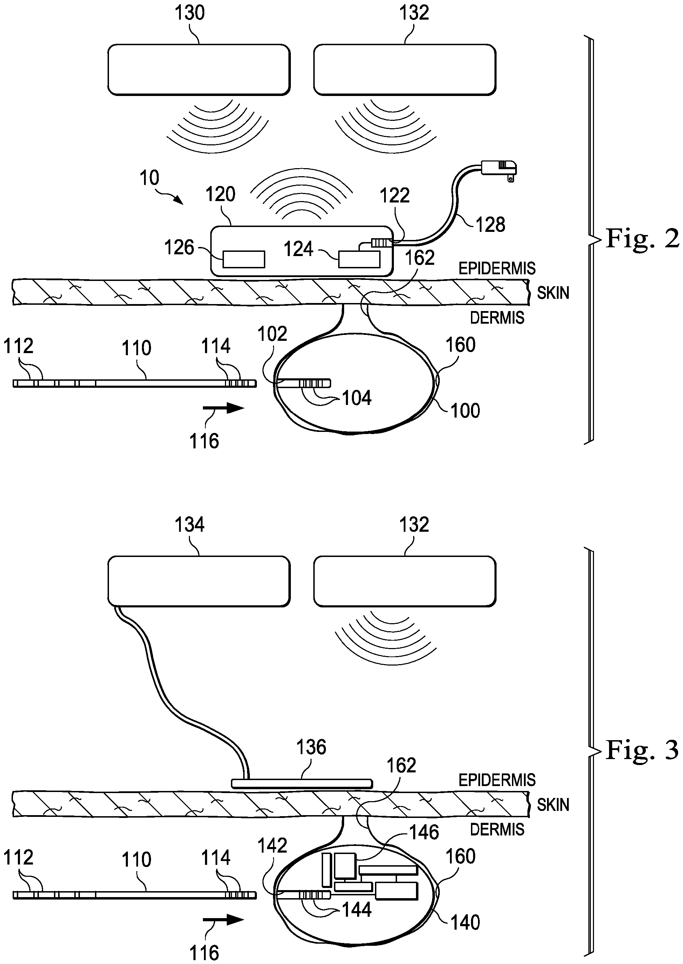

Referring now to Fig. 2, there is shown a first portion of a modular stimulation system 10 according to a first aspect of the invention. The stimulation system 10 includes an implanted passive receiver (PR) 100 interconnected with a stimulation lead 110. The stimulation lead 110 has a proximal end portion that is sized to be received within socket 102 on the passive receiver 100 by moving the lead in the direction of arrow 116 until the proximal end of the lead 110 is fully seated in the socket 102. It will be understood that the PR may include a releasable locking mechanism to securely retain the lead within socket 102. The proximal end includes a series of contacts 114 that are configured to mate with contacts 104 positioned within socket 102. Contacts 114 are electrically connected to stimulation electrodes 112 disposed at the distal end portion of lead 110. In the illustrated embodiment, lead 110 is substantially flexible thereby allowing the lead to bend along its length and accommodate a variety of placement positions of the electrodes 112 in relation to the passive receiver 110. As shown in Fig. 1, the passive receiver 100 is passed through an access channel 162 in the skin and dermis into an implantation pocket 160 formed inside the patient to hold the passive receiver. After implantation, as shown in Fig. 2, the skin is closed such that no portion of the passive receiver 100 or lead 110 extends through the skin.

The passive receiver 100 includes internal electronics to enable the device to receive external power and controls enabling the receiver to energize the stimulation electrodes in at least one stimulation configuration. In the illustrated embodiment, the passive receiver 100 does not include an internal power supply sufficient to perform electrical stimulation. Instead, an external pulse generator (EPG) 120 transcutaneously transmits both power and controls instructions through the patient's skin to the passive receiver 100 to thereby control stimulation in the stimulation electrodes 112. The illustrated EPG 120 includes a socket 122 configured to receive power from a power source, such as for example, from a cord 128 connected to a wall charger. The socket 122 includes electrical connections that deliver power to a rechargeable battery 124 positioned inside the EPG. The EPG may operate on battery power or wall power. The EPG includes a coil 126 to transmit power and/or information to the passive receiver implanted in the patient. It will be appreciated that in a typical application, the EPG will be maintained in a stationary position relative to the PR for an extended period of time to provide the patient with electrical stimulation of nerves adjacent the electrodes 112. In an alternative form, a sending/receiving power and communication coil (such as shown in Fig. 3) may be held in position on the skin while interconnected by flexible wires to the EPG unit. In one example embodiment, the EPG is programmable by either a patient programmer 130 or a clinician programmer 132. Although not illustrated in Fig. 2, the EPG includes communications hardware, memory, and one or more processors. Further, the EPG may include an accelerometer to record patient activity. These components operate to communicate with the external programming devices as well as operating to transmit stimulation signals and power to the passive receiver 100.

In one aspect, the embodiment illustrated in Fig. 2 is understood to form stage one of a multi-stage implantation process for neurostimulation. The passive receiver 100 includes a very limited number of simple electrical components making it a relatively inexpensive device in comparison to a traditional implantable pulse generator having an internal power supply. Traditional implantable pulse generators are expensive for a variety of reasons. Some of the reasons for the expense include the complexity of the internal circuitry as well as the use of an internal power source that must power the stimulator for significant periods of time. In addition to the cost of developing and manufacturing the IPG, extensive testing is required to confirm that the device will operate as intended for several years without failure and that the battery will both operate as intended and not leak harmful chemicals into the patient. The present passive receiver avoids these costs and expenses by providing a simplified system that includes only a power/data receiving coil, and minimal internal circuitry needed to transform the power to stimulation pulses. As a result, the sales price of the passive receiver is expected to be less than one half of the sales price of a traditional implantable pulse generator. In further features, the sales price of the passive receiver is expected to be less than one third of the sales price of the implantable pulse generator and can be as low as one fifth to one tenth of the sales price of the implantable pulse generator.

Referring now to Fig. 3, there is shown one form of a second stage, or stage two, of a modular neurostimulation system. The lead 110 is substantially the same as the lead 110 shown in Fig. 2. In many circumstances, lead 110 of Fig. 1 will remain in position within the patient, and as explained further below, the passive receiver 100 will be replaced with an implantable pulse generator (IPG) 140. The implantable pulse generator 140 includes a socket 142 adapted to receive the contacts 114 on the proximal end of the lead when it is urged in the direction of arrow 116 into socket 142 until the contacts 114 are aligned with contacts 144. It will be understood that the IPG may include a locking mechanism to securely retain the lead within socket 142. The IPG 140 includes internal circuitry and a power source, such as a rechargeable battery, allowing the IPG to independently stimulate lead electrodes 112. In addition internal circuitry includes communication hardware allowing it to communicate through the skin once positioned in pocket 160 through access path 162. In addition, in the illustrated embodiment, the IPG 140 includes a rechargeable battery and at least one coil to inductively couple to an external power source. As is known from conventional stimulation systems, IPG 140 may be re-supplied with power and controlled by external devices communicating through the skin. Specifically, as shown in Fig. 3, a patient programmer/recharger module 134 may communicate control instructions as well as transmit power through the skin using the recharging/programming coil 136. As a further feature, a clinician programmer 132 may wirelessly communicate with the IPG to control stimulation settings and make other adjustments to the IPG.

In one aspect, the passive receiver 100 has a displacement volume that approximates the displacement volume of the IPG 140. It will be appreciated, that as the tissue of pocket 160 heals around the passive receiver it will substantially match the displacement volume of the passive receiver. Thus, having a passive receiver 100 with a displacement volume that substantially matches the displacement volume of the IPG 140 greatly simplifies the surgical procedure of explanting the PR and implanting the IPG. The modular staged implant system of the present disclosure contemplates that the passive receiver displacement volume is greater than 65% of the displacement volume of IPG 140. In one aspect, the displacement volume of the passive receiver is greater than 75% of the displacement volume of the IPG 140. In still another aspect, the displacement volume of the passive receiver is greater than 90%, and preferably greater than 95%, of the displacement volume of the IPG 140. In still a further feature, the displacement volume of the passive receiver is the same as the displacement volume of the IPG 140. Still further, as explained in more detail below, the outer perimeter of the passive receiver may be enhanced to create a displacement volume that substantially matches the displacement volume of the IPG that forms the second stage of the modular implant system.

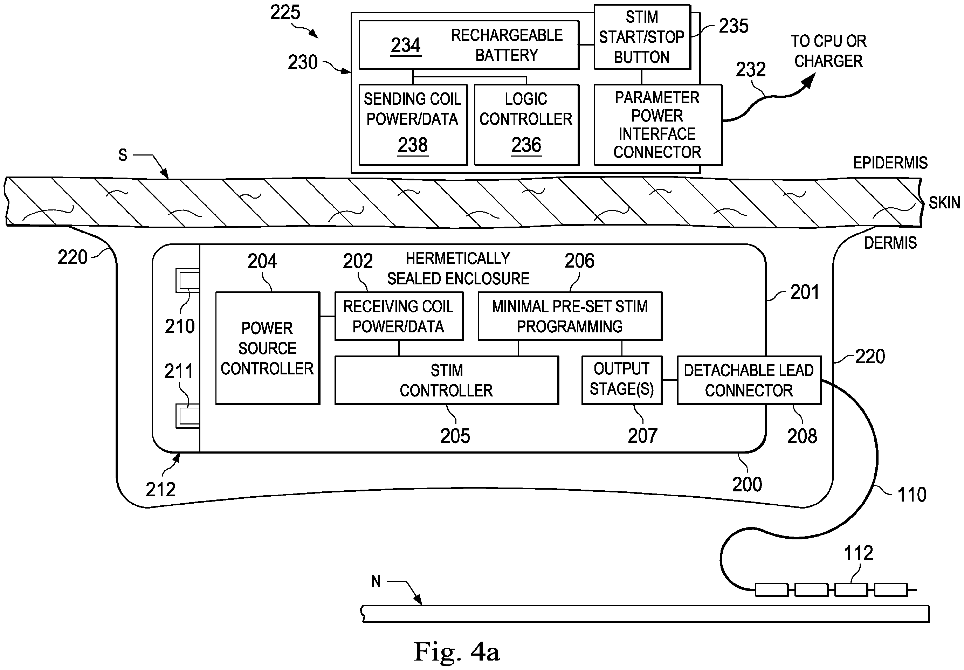

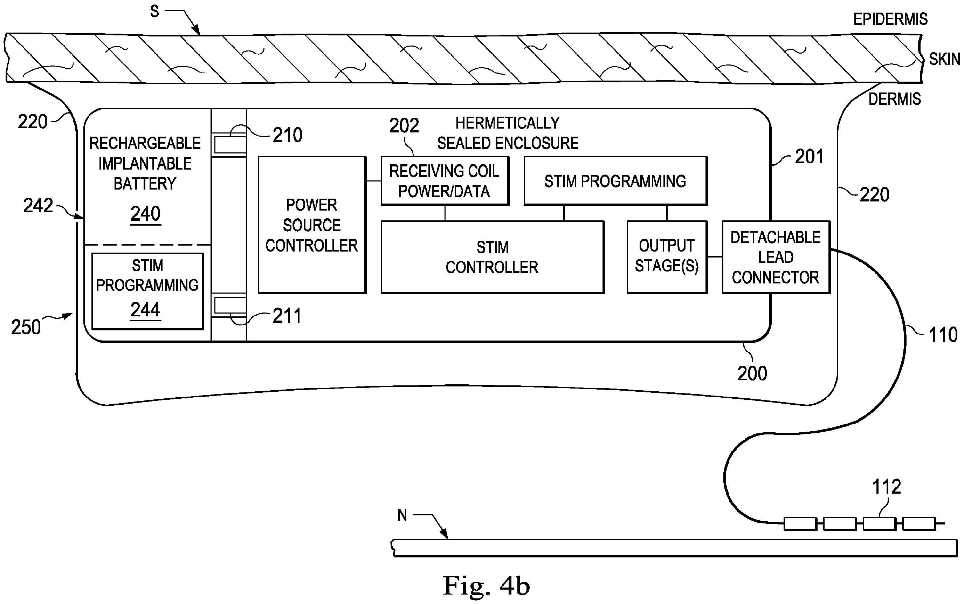

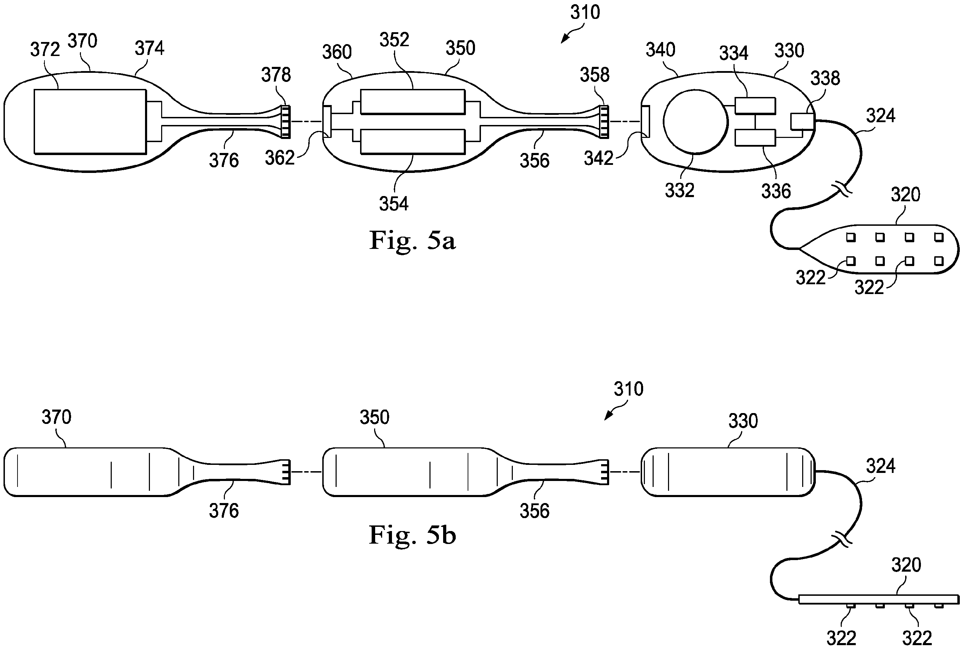

Referring now to Figs. 4a and 4b, there is shown a further embodiment of a modular stimulation system 225 according to another aspect of the invention. Expandable passive receiver (EPR) 200 is illustrated implanted beneath skin S in pocket 220. In the illustrated embodiment, passive receiver 200 includes a power/data receiving coil 202 and a power source controller 204. In addition, the passive receiver includes a stimulation controller 205 operating on a minimal amount of pre-set stimulation programming parameters stored in memory 206. These pre-set stimulation programming parameters may include amplitude, polarity, pulse width, frequency, or a combination of all the above stored as individual programs that may be controlled by an external pulse generator. The passive receiver provides stimulation pulses through the output stages 207 to the lead connector assembly 208 located on the distal end of the passive receiver. As illustrated, a lead 110 is coupled to the lead connector assembly 208. In one aspect, the lead is detachably connected to the passive receiver by a detachable coupling mechanism in the connector assembly 208. In an alternative aspect, the lead 110 is pre-assembled with the passive receiver 200 creating an integral, single piece unit that may be implanted in the patient without making any connections. Regardless of the connection between the lead and the passive receiver, the lead stimulation electrodes 112 will be position adjacent to a nerve N sufficiently close to stimulate the nerve with electrical pulses. In a further feature, it is contemplated that the lead may include more electrodes than the passive receiver is configured to stimulate. For example, the stimulation system may utilize an eight electrode paddle lead such as shown in Fig. 5a, while the passive receiver is only configured to stimulate at most four electrodes. In this manner, it is possible to trial a small subset of the electrodes in a trialing configuration to determine if any efficacy exits. If efficacy is present, as explained further below, an implantable pulse generator configured with greater stimulation capability may be implanted to utilize more of the available electrodes.

The EPR 200 includes a hermetically sealed enclosure 201 with three connectors extending through the enclosure. The first connector is the lead wire connector 208 previously described. In addition, a power connector 210 and a data connector 211 also extend through the enclosure to the exterior of the passive receiver on its proximal end. In the embodiment of Fig. 4a, a cap 212 is attached to the enclosure to cover the power connector 210 and the data connector 211. As illustrated in Fig. 4a, the cap has external dimensions that closely match the external perimeter of the enclosure 201 at the proximal end of the passive receiver. As a result, the cap 212 inhibits contamination of the connectors 210, 211 by bodily fluids and tissues, and also presents a smooth outer surface in combination with the enclosure to inhibit tissue irritation. In a further feature, cap 212 has an expanded outer geometry substantially matching the outer geometry of second stage components (discussed below) that may be added to the power connector 210 and/or data connector 211 to create an expanded device. It will be appreciated that when this feature is utilized, the displacement volume of the passive receiver exterior enclosure 201 in combination with cap 212 will substantially match the displacement volume of the stage two implantable pulse generator discussed below. In one aspect, the exterior of enclosure 201 is smooth to inhibit tissue irritation and allow surrounding tissues to slide smoothly across the surface. This may be particularly advantageous in peripheral nerve stimulation applications where the adjacent muscles will be contracting and relaxing during patient movement. Still further, in a further aspect, the exterior of enclosure 201 may include a lubricious coating to further reduce friction. In an alternative approach, the exterior surface of enclosure 201 may include surface roughening to engage adjacent tissue and inhibit movement therebetween. In the alternative or in conjunction with the surface roughenings, the surface may also include a tissue ingrowth coating that tends to promote the growth of tissue fibers that adhere to the surface of the enclosure. For example, one coating may include aluminum trioxide or collagen.

The expandable passive receiver 200 is powered and controlled by an external pulse generator (EPG) 230. The EPG includes a connection 232 to a programmer and/or charger to supply programming information to the EPG and/or provide power to the rechargeable battery 234. The EPG also includes a logic controller 236 that controls information and power transmitted/received through coil 238 to and from the passive receiver 200.

Referring now to Fig. 4b, there is shown a second configuration of the expandable passive receiver 200 forming a second stage of a modular stimulation system. As illustrated, the EPR 200 remains in tissue pocket 220 with lead 110 connected and the electrodes 112 positioned adjacent nerve N. The lead 110 remains attached to the EPR 200, however, cap 212 has been removed and replaced with a power/programming module 240. The addition of the power/programming module 240 to the passive receiver 200 creates a modular implantable pulse generator (MIPG) 250 as shown in Fig. 4b. The power/programming module 240 is hermetically sealed and includes an implantable battery 242 connected to power connector 210 and a stimulation programming module 244 connected to data connector 211. In the illustrated embodiment, the implantable battery 242 is a rechargeable battery. The battery 242 is recharged with power received at the power receiving coil 202 of the passive receiver. Charging of the battery can be controlled by the power source controller 204 or by control systems integrated into the power/programming module 240. Still further, powering and operation of the passive receiver 200 may be continued via an EPG as set forth above with respect to the system of Fig. 4a. Specifically, if battery 242 or stimulation programming module 244 become in operable, the passive receiver components of the MIPG 250 can still be operated to provide neurostimulation pulses to the leads 112 positioned adjacent nerve N. It is also contemplated that the battery 242 and/or the stimulation programming module of the EPR may be single use items that can be replaced when the non-rechargeable battery is depleted and/or the stimulation programming module needs to be updated.

A further modular neurostimulation system 300 according to the present disclosure is shown in Figs. 5a and 5b. The modular neurostimulation system 300 includes a passive receiver 330, an implantable pulse generator controller 350 and an implantable power supply module 370. The passive receiver 330 includes a power/data receiving coil 332 interconnected with a stimulation controller 334, an output stages component 336 and a coupling assembly 338. A housing 340 surrounds the electrical components to form a generally flat ovoid shaped device that, as shown in Fig. 5a, has a length greater than a width, both of which are greater than the thickness shown in Fig. 5b. In one aspect, the material forming housing 340 is substantially flexible giving the passive receiver greater flexibility when implanted in the body. In the illustrated embodiment, a paddle lead 320 having eight electrodes 322 is connected to the distal end of the passive receiver via lead wires 324 to the coupling assembly 338 via permanent attachment such that the lead wire cannot be removed from the passive receiver without destruction of the passive receiver. As explained above, it is also contemplated that in a further embodiment the lead wires 324 may be releasably coupled to the coupling assembly 338. A coupling recess 342 is formed in the proximal end of the passive receiver.

The implantable pulse generator controller 350 includes at least a stimulation controller 352 and a memory module 354 operable to generate stimulation pulses in accordance with stored programming parameters. A housing 360 surrounds the electrical components to form a generally flat ovoid shaped device that, as shown in Fig. 5a, has a length greater than a width, both of which are greater than the thickness shown in Fig. 5b. In one aspect, the material forming housing 360 is substantially flexible giving the passive receiver greater flexibility when implanted in the body. The IPG controller 350 includes a flexible lead 356 with a coupling assembly 358 configured for releasably mating with coupling recess 342 on the passive receiver 330. In one mode of operation, the IGP controller 350 is coupled to the passive receiver 330. The flexible lead 356 allows the components to positioned at a variable distance and angle with respect to each other. The system is operable with just the passive receiver 330 and implantable pulse generator controller 350. The passive receiver receives power through the power coil 332 which is used to power the IPG controller 350 and provide stimulation power to the electrodes under control of the IPG controller.

In a further aspect, the modular neurostimulation system 310 includes a power supply module 370 that can be coupled to the IPG controller 350. The power supply module 370 includes a battery 372, a flexible lead 376 and a coupling assembly 378 configured for releasably mating with coupling recess 362. As discussed above, a flexible material surrounds the battery to form a generally flat ovoid housing 370.

Thus, although the components of Figs. 5a and 5b are shown extending along a longitudinal axis, the flexible leads allow the components to be positioned at an angle with respect to the longitudinal axis. Further, the components are sized substantially smaller than a traditional IPG such that when positioned in the patient near the skin in limb peripheral nerve stimulation applications, the patient is less likely to be irritated by a large can type enclosure associated with a traditional IPG and the individual components may move more easily under the skin. In one aspect, each of the modules is approximately the size of a U.S. quarter.

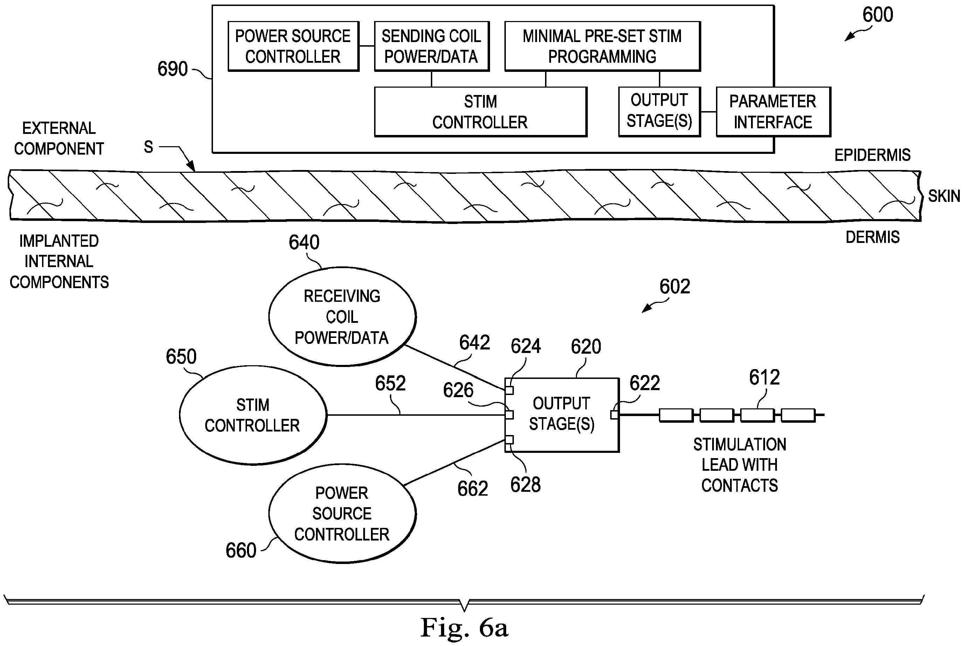

Referring now to Figs. 6a-c, there is shown a further embodiment of a modular stimulation system 600 according to another aspect of the invention. In a first form, expandable passive receiver (EPR) 602 is illustrated implanted beneath skin S. In the illustrated embodiment, the EPR 602 includes a central module 620 referred to as the output stages component. The central module 620 includes at least one output port having a lead coupling 622 for releasable coupling with a lead 612 as disclosed and described in more detail above. Although only a single output port lead coupling is illustrated, it is contemplated that the central module 620 may include two or more lead couplings permitting the output stages to electrically stimulate more than one lead array. In the illustrated embodiment, the central module 620 includes at least three expansion ports 624, 626 and 628. As illustrated, a receiving coil power/data module 640 may be connected to the expansion port 624 by a flexible wire or other electrically conductive member 642. The receiving coil module 640 includes a conductive coil that can be inductively coupled with an external pulse generator 690. The EPG 690 can be configured to substantially match the EPG 230 of Fig. 4a. As shown in Fig. 6a, the EPG 690 includes a power source controller, a coil for sending power and data to the EPR 602, a memory having minimal preset stimulation programming, output stage(s), stimulation controller module and a parameter interface module that can communicate with a further device to receive stimulation parameter information and output information from the EPG.

The receiving coil module 640 of the EPR 602 includes only minimal electrical components to reduce its external dimensions and overall size. In the illustrated embodiment, the receiving coil module 640 is generally cylindrical and has a diameter of less than 2.5 centimeters. In a preferred aspect, the coil module diameter would be less than 1.5 centimeters. Still further, in one configuration, the wire 642 has a length of about 10 centimeters or that is approximately four times the diameter of the coil module 640. In another aspect, the wire 642 has a length of less than 7 centimeters. As shown in Fig. 6a, the EPR 602 further includes stimulation control module 650 connected to expansion port 626 via wire 652 and power source control module 660 connected to expansion port 628 via connector 662. The stimulation control module 650 and power source control module 660 are operatively coupled to and in communication with the output stages module 620 to thereby received power and/or programming information through the receiving coil 640 and transform the received signals into stimulation signals of the desired form that are output through coupling 622 to the lead 612. As will be appreciated, the EPR 602 has four electrical components, each having individual external housings, connected via insulated wires extending through body tissue to form a distributed passive receiving system. Each of the stimulation and power source control modules 650 and 660 may have dimensions similar to module 640 and be coupled to the output stages module 620 via connectors having lengths between 2 and 15 centimeters. In one aspect, modules have different dimensions with at least one of the modules have a maximum dimension less than 1 centimeter. In one aspect, each of the connectors 642, 652 and 662 have different lengths such that when fully extended along the longitudinal axis of the output stages module 602 the remaining modules are spaced along the longitudinal axis a different amount such that there is no longitudinal overlap between each of the modules. In this configuration, the modules resemble a string of beads with each module having a different longitudinal position. As a result, the modules or beads 640, 650 and 660 may be spaced apart within the patient to reduce the overall configuration to a series of small bumps under the patient's skin. As will be appreciated, the configuration of the skin, fat, muscle and bones of the limbs often leave few large areas that can easily receive a large implant, such as a traditional IPG, that will not be uncomfortable to the patient and not be significantly noticeable through the skin. The distributed modules or beads of the present embodiment allow positioning each module in the desired location to avoid patient discomfort and to minimize disfiguring of the limbs of the patient by unsightly bulges showing through the skin.

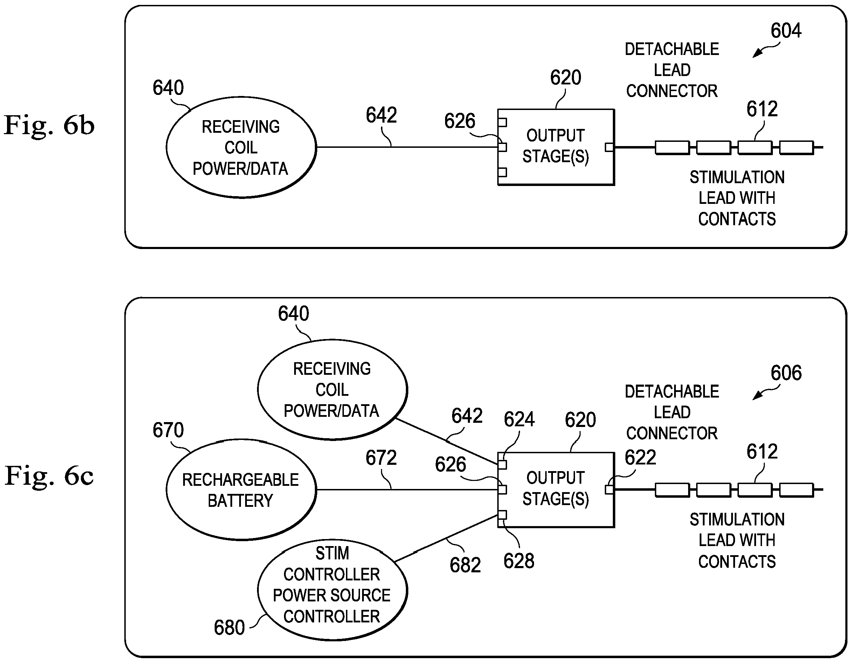

Referring now to Fig. 6b, there is shown a minimalist design of a EPR 604. The EPR 604 includes only an output stages module 620 interconnected with a receiving coil module 640 via connector 642 coupled to expansion port 626. In this configuration, the power and stimulation controls are delivered by the EPG to provide stimulation signals to the lead 612. Either of the EPR 602 or 604 may be expanded to form a modular implantable pulse generator MIPG 606 as shown in Fig. 6c. The output stages central module 620 includes a receiving coil module 640 connected to expansion port 624, a rechargeable battery 670 connected to expansion port 626 via connector 672 and a combined power source and stimulation controller 680 connected to expansion port 628 via connector 682. In operation, the stimulation controller module 680 controls the output stages module 620 to deliver the programmed stimulation signals powered by battery module 670 to the lead 612. Power to recharge the battery is periodically received by the receiving coil 640. Similarly, the MIPG 606 may send and receive data through the coil module 640.

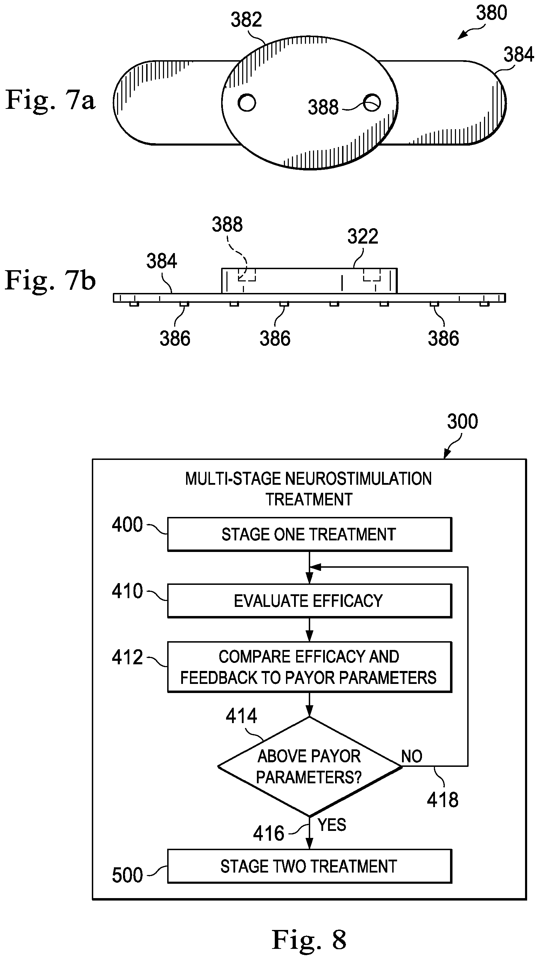

Referring now to Figs. 7a and 7b, there is shown an integrated neurostimulation system 380 according to another aspect of the present disclosure. The integrated system includes a passive receiver 382 directly joined to an electrode array 384 without a lead extending away from the perimeter of the electrode array. The passive receiver 382 includes a pair of couplings 388 that are adapted to receive and retain an implantable pulse generator. The electrode array includes sixteen electrodes 386. The integrated neurostimulation system 380 can be utilized for stage one nerve stimulation by external control and powering of the passive receiver. If an implantable pulse generated is desired, it can be mounted to the couplings 388 to provide a more permanent power source and control system to thereby form an expanded internal pulse generator.

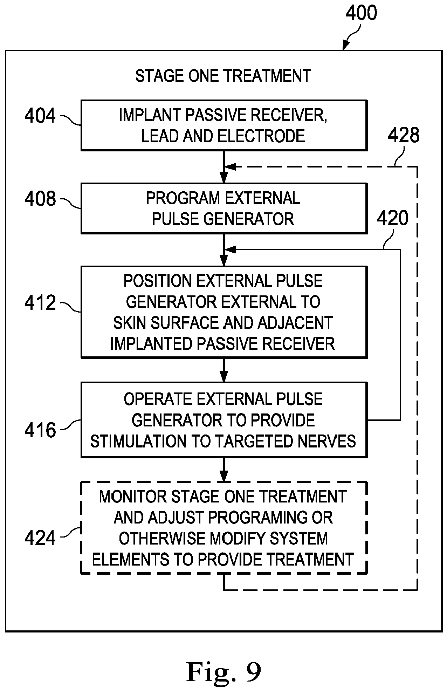

Referring now to Fig. 8, a flowchart is shown illustrating an embodiment of a multi-stage neurostimulation treatment 300 method. More particularly, multi-stage neurostimulation treatment 300 includes stage one treatment 400 involving the implantation of a passive receiver with leads extending to electrodes placed adjacent at least one nerve to be stimulated. The detailed steps of the stage one stimulation method are explained in greater detail with respect to Fig. 9. After the stage one implantation has occurred, the method continues at step 410 with an evaluation of the efficacy of the neurostimulation by the passive receiver. The patient and healthcare provider can evaluate the effectiveness and make adjustments to stimulation intensities, polarities, electrode patterns, pulse widths, etc. to attempt to achieve maximum pain relief. In addition, the method includes comparison of the patient efficacy in step 412 to payor parameters in step 414 to determine if the patient is eligible to be reimbursed for permanent implantation of an implantable pulse generator. More specifically, certain payors such as insurance companies, Medicare, etc. may set specific benchmarks which must be obtained before the patient is eligible for coverage of a further surgical procedure and cost of an IPG. For example, the payors may require a certain percentage of pain relief, reduction in narcotic medication usage or a significantly increased activity level before agreeing to reimburse the patient for the IPG. To assist in determining efficacy, in one aspect, the EPG records stimulation data concerning the following parameters:

1) Duration of stimulation per day. This provides some indication that the patient is in fact using the stimulation device as directed and how long each stimulation session lasts.

2) Stimulation program selected by user, including patient initiated program changes during the day. This provides feedback concerning whether a single simulation program or intensity is providing patient relief, or if the patient must continually make modifications to program setting to attempt to gain relief which may be an indication that the patient is not suitable for a permanent IPG or that changes concerning lead placement or stimulation programming need to be performed in the stage one process before proceeding to the stage two process.

3) Total length of stimulation time during the stage one implantation. There may be extended periods of days when a patient neglects to perform stimulation, likely because the pain is less severe, or the patient receives pain relief using other methods. This provides an indication of whether a patient's pain levels warrant a permanent IPG or if the patient may be best served by intermittent use of the passive receiver implanted in stage one.

4) Patient pain levels. In one aspect, the EPG and/or related computer software may include prompts to the patient to record pain levels during times of stimulation and at times when stimulation is not being applied. In addition to or as an alternative, the patient may be interviewed to evaluate their feedback on the efficacy of the neurostimulation. This information may be overlaid with the information above to help evaluate stimulation efficacy. Still further, the EPG may include an accelerometer to gauge the patient activity levels during periods of stimulation. This data may be used directly as an indicator of patient pain relief. Typically, patients with pain have much less limb movement than those with reduced pain. In a further aspect, the patient may were an accelerometer for a period of time before implantation of a passive receiver. The pre-implantation data can then be compared to the movement information obtained during stimulation.

5) Patient side effects. In one aspect, the EPG and/or related computer software may include prompts to the patient to record side effects during times of stimulation and at times when stimulation is not being applied. In addition to or as an alternative, the patient may be interviewed to evaluate their what, if any, side effects they are experiencing during neurostimulation. This information may be overlaid with the information above to help evaluate stimulation efficacy.

In one aspect, the patient programmer and/or an associated computer are utilized to gather data on one or more of the parameters above. The healthcare provider and/or payor may have a specific range of individual benchmarks within each parameter and/or may incorporate the various parameters into a composite score. The score may be used to indicate if the patient is an acceptable candidate for a permanent IPG. In one aspect, the patient programmer periodically communicates patient data to the physician and/or payor. Based on the collected information the physician may make a determination that an IPG would be beneficial and the payor may determine if coverage is available for an IPG implantation procedure. If the patient efficacy satisfies the benchmark requirements, the method continues to a stage two treatment and method at step 500.

Referring now to Fig. 9, additional detail is provided of stage one treatment 400. At 404, the passive receiver 100 is implanted together with the lead 110 and the stimulation electrodes 112. The passive receiver 100 is preferably implanted in a location below the patient's skin that is suitable for possible future implantation of an implantable pulse generator, such as IPG 140. As shown in Fig. 2, the implantable components may be passed through access path 162 and into a pocket 160. At 408, the EPG 120 is programmed such as by using an EPG patient programmer 130 or clinician programmer 132 configured to communicate to the EPG 120. At 412, the EPG 120 is positioned external to the skin surface and adjacent the implanted passive receiver 100. At 416, the EPG 120 is operated to send power and stimulation signals to the passive receiver and thereby stimulate electrodes 112 via lead 110.

The stage one treatment 400 offers the advantage that use of the EPG 120 is controlled by the patient. The EPG may be set aside for some time, such as for the patient to undertake certain activities, including bathing or swimming. Still further, the EPG may be used when patient pain levels are intense and discontinued when pain levels are bearable. Further, the EPG 120 may even be replaced if it is damaged, lost, becomes inoperable, needs a new battery, or the programming needs to be upgraded. The operation of the EPG continues in loop 420 between steps 412 and 416 for as long as the patient receives a benefit. The use of the EPG in this mode of operation may continue indefinitely.

At optional step 424, the results of the stage one stimulation provided by the EPG 120, passive receiver 100, lead 110 and electrodes 112 are monitored. Follow-up treatment at 424 during the stage one treatment 400 may include adjustment of the programming provided to the EPG 120 and/or adjustment of the location of the electrodes 112. Accordingly, at loop 428, the EPG 120 may be reprogrammed to adjust the stimulation signal sent to the electrodes 112. In addition, as noted above, the treating physician and/or the patient may use optional remote controllers 130 and 132 to adjust one or more tunable parameters associated with the EPG 120.

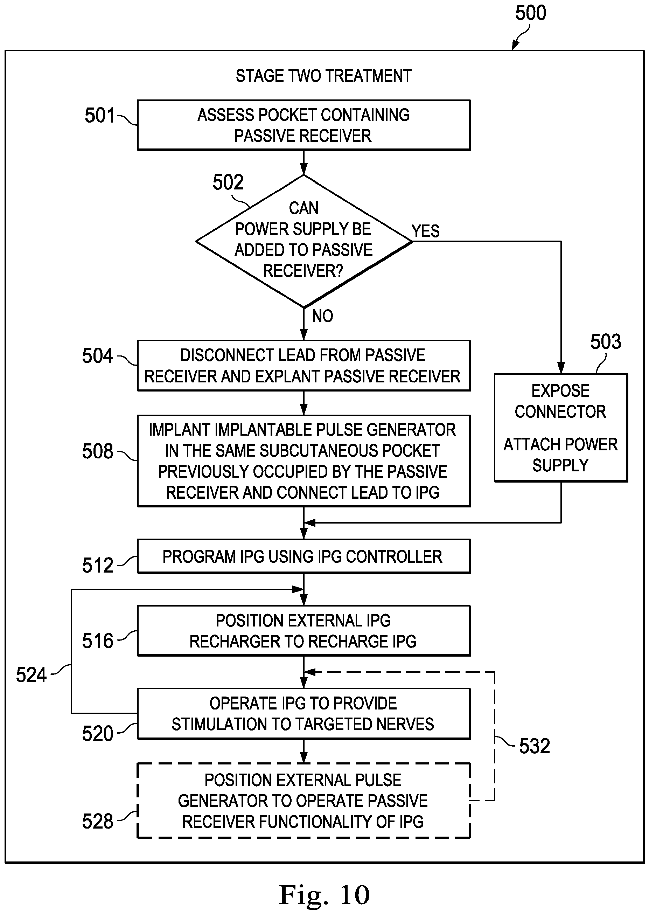

As mentioned above with respect to Fig. 8, if the passive receiver and EPG are providing the patient with sufficient pain relief, a permanent IPG may be implanted to provide an internally powered and controlled pulse generator to stimulate the implanted stimulation electrodes. Referring now to Fig. 10, additional detail is provided of a stage two treatment 500. At step 501 the healthcare provide accesses the tissue pocket containing the passive receiver. At step 502, the healthcare provider determines if the passive receiver is an expandable passive receiver that may be adapted to receive additional components. If yes, the method proceeds to step 503 where the physician exposes the connections for a power/programming module such as module 240 discussed with respect to Fig. 4b or modules 350 and 370 discussed with respect to Fig. 5a. The power/programming module is then electrically coupled to the expandable passive receiver to form a modular implantable pulse generator (MIPG). If the passive receiver is not expandable, the method continues to step 504 where the lead is disconnected from the passive receiver and the passive receiver is removed from the patient. A self contained implantable pulse generator (IPG) is then coupled to the lead and the IPG is implanted in the patient. In one aspect, the IPG is implanted into the same tissue pocket previously occupied by the passive receiver as set forth in step 508. As noted above, an advantage of at least one embodiment of the present invention is that the IPG 140 occupies substantially the same space, including shape and volume, as the passive receiver 100. Such similar sizing enables the surgeon to perform the implantation procedure to remove the passive receiver 100 and implant the IPG 140 in a relatively short period of time. Advantageously, the procedure to implant the IPG 140 would likely be conducted on an out-patient basis, thereby reducing procedure costs and enabling greater medical insurance coverage for the possible population of patients possessing indications for a multi-stage neurostimulation system.

After placement of either a MIPG or a self contained IPG, the implanted pulse generator is programmed in step 512, such as by using an IPG controller that is configured to wirelessly communicate with the IPG. At 516, an IPG recharger 166 is positioned proximate the IPG 154 for externally recharging the IPG 154. That is, in at least one embodiment, the IPG recharger 166 uses induction to recharge the power cell residing within the IPG 154. In a preferred aspect, the stimulation control data gathered from use of the EPG and passive receiver in stage one is used to program the IPG. In still a further aspect, the IPG or power/programming module is programmed outside the body before implantation with one or more stimulation protocols substantially matching the EPG stimulation protocols applied in stage one. In this manner, programming the IPG can be substantially faster than in traditional IPG placements where the system must be customized based on patient feedback after implantation.

Use of the IPG or MIPG continues in a traditional manner. Specifically, at 520, the IPG 140 or MIPG 250 is operated to provide a stimulation signal to the electrodes 112 via lead 110. At loop 524, the IPG recharger 136 is used to again to recharge the IPG 140 or MIPG 250.

As explained above, the MIPG 250 retains the components of the passive receiver 200 in an operable manner such that the passive receiver may be operated by an external pulse generator if desired. In at least one embodiment, although not required for all embodiments, the IPG 140 may optionally incorporate a "back-up" passive receiver. Here, the back-up passive receiver is similar in functionality to the passive receiver 100 of the stage one system elements and as used in the stage one treatment described above. More particularly, the IPG 140 may include a passive receiver enabling the IPG 140 to function as a passive receiver should the IPG 140 cease to function due to either a low battery condition, unavailability of an IPG recharger, or because the power cell in the rechargeable IPG 140 is no longer capable of being recharged. Accordingly, should the IPG 140 or MIPG 250 no longer be capable of recharging or is otherwise temporarily inoperable, an external pulse generator could be positioned adjacent the IPG 140 or MIPG 250. At loop 532, the external pulse generator is then used at 520 to operate the passive receiver built-in to the IPG 140 or MIPG 250, thereby providing stimulation power and a stimulation signal to the electrodes 112 that remain to lead 110. Therefore, at 528, an external pulse generator is optionally positioned near the IPG 140 or MIPG 250 to operate the passive receiver and provide neurostimulation to the patient.

The stage one system elements of Figs. 2 and 4a allow the treating physician to assess the efficacy of the neurostimulation system before undertaking the cost and expense of implanting a permanent IPG system. Therefore, health insurance companies can request use of a staged neurostimulation system including stage one system elements, and during such treatment receive a physician's report as to efficacy of the stage one stimulation before authorizing implantation of an IPG and incurring the costs associated with the IPG and its placement. From the physician's and the patient's perspective, health insurance reimbursement support for the stage two treatment 500 can be secured in advance without stopping treatment because ongoing treatment is available to the patient by using the stage one system elements 100. In addition, since the stage one system elements do not include elements that extend through the patient's skin, the risk of infection from an open wound around an electrode lead is avoided. Furthermore, the trialing functionality of stage one treatments may extend for many weeks or months without increasing the risk of infection.

In addition to the foregoing advantages, stage one treatment 400 also enables use of a variety programs and testing associated with one or more EPGs 120. For example, with extended stage one treatment 400, the physician can test a variety of signal strengths, pulse widths and electrode configurations and continue to modify and vary the treatment plan to achieve improved results. In addition, the limited and relatively minimally invasive nature of the stage one implantable elements means that the stage one treatment 400 can be easily suspended if the patient happens to experience an unrelated but sufficiently serious medical condition, such as physical trauma. Such extended testing available from the stage one treatment 400 can help in eliminating false positives resulting from a placebo effect, adaptation, plasticity, and/or secondary gain sometimes observed during shorter-term efficacy testing.

For those situations wherein stage two is indicated, steps are readily undertaken to modify the stage one system to the level of a stage two system as described herein. On the other hand, for those stage one systems that do not appear sufficiently successful (or otherwise do not warrant taking to stage two), three possibilities exist going forward. These include: (a) removing the stage one implantable elements 116 and terminating the stage one treatment 400 in its entirety; (b) modifying the stage one treatment 400 in some manner to improve its effectiveness, and thereafter continuing to evaluate the efficacy of the modified stage one treatment; or (c) simply leaving the stage one implantable elements in place and continue to accept the results as experienced by the patient. Indeed, the stage one treatment 400 may be continued indefinitely. That is, ongoing treatment to the patient using an EPG 120 can continue as a relatively inexpensive solution if the patient chooses to never proceed with stage two treatment 500, or if the patient cannot afford the stage two treatment 500, particularly if the patient cannot obtain insurance approval for coverage for substantial reimbursement of the stage two treatment 500.

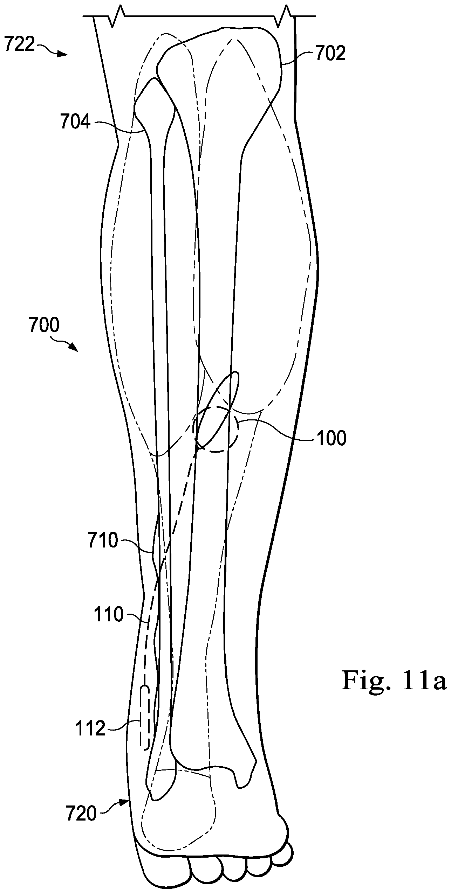

Referring now to Fig. 11a, and in accordance with at least one embodiment, the stage one implantable elements of Fig. 2 are shown implanted in the lower leg 700 of a patient. More particularly, a passive receiver 100 has been implanted near the calf muscles, with an implanted lead 110 extending to implanted electrodes 112 positioned for stimulation of the posterior tibial nerve 710. All stage one implantable elements reside below the knee and are contained within the length of the tibia 702 and the fibula 704. That is to say that lead 110 does not traverse a joint as it extends between the passive receiver 100 and the electrode 112. Still further, none of the components of the stimulation system extend across or into a joint. The implant system shown in Fig. 11a is positioned between the knee joint 722 and the ankle joint 720.

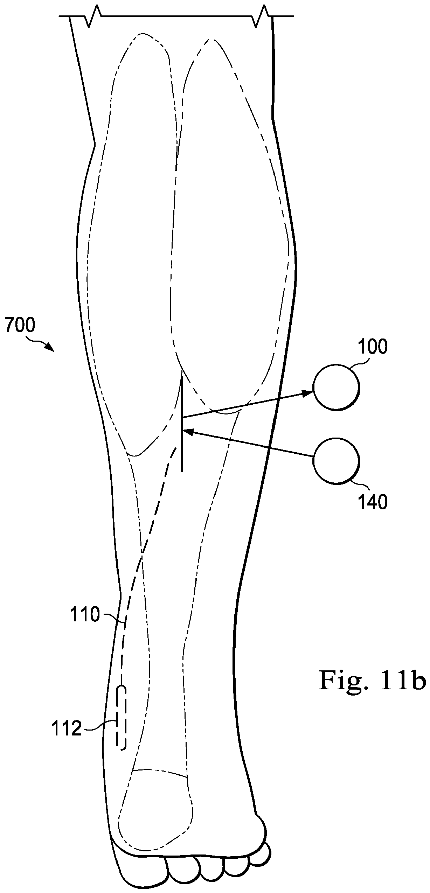

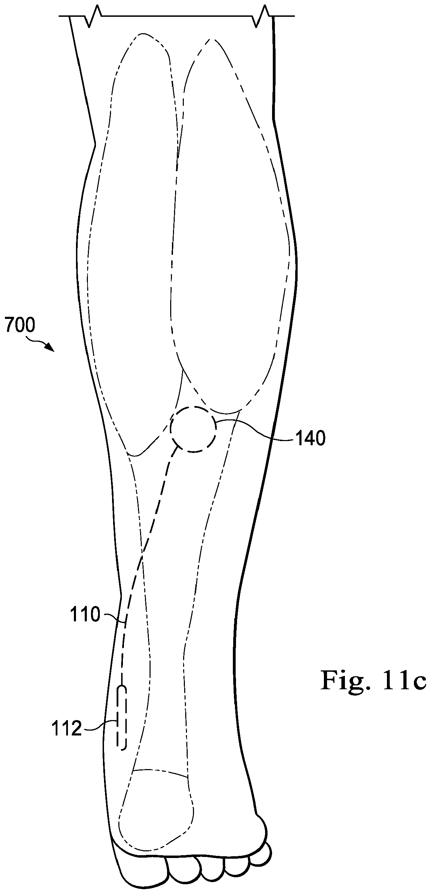

At some time after implantation of the stage one implantable elements and operating the stage one system elements in accordance with steps 412-420 of the stage one treatment 400, the treating physician and the patient may decide to move forward with stage two treatment 500. With reference now to Fig. 11b, the explantation of the passive receiver 100 and the implantation of the IPG 140 is shown. As explained above, the passive receiver 100 is removed from the first tissue pocket and after connection of the lead 110 the IPG 140 is implanted into the first tissue pocket. As explained above, as an alternative for stage one embodiments shown in Fig. 4a, the passive receiver is left in place and a power and programming module is added thereto to form a modular implantable pulse generator. Thereafter, as depicted in Fig. 11C, the stage two implantable elements reside within the patient, and include the IPG 140, lead 110 and electrodes 112.

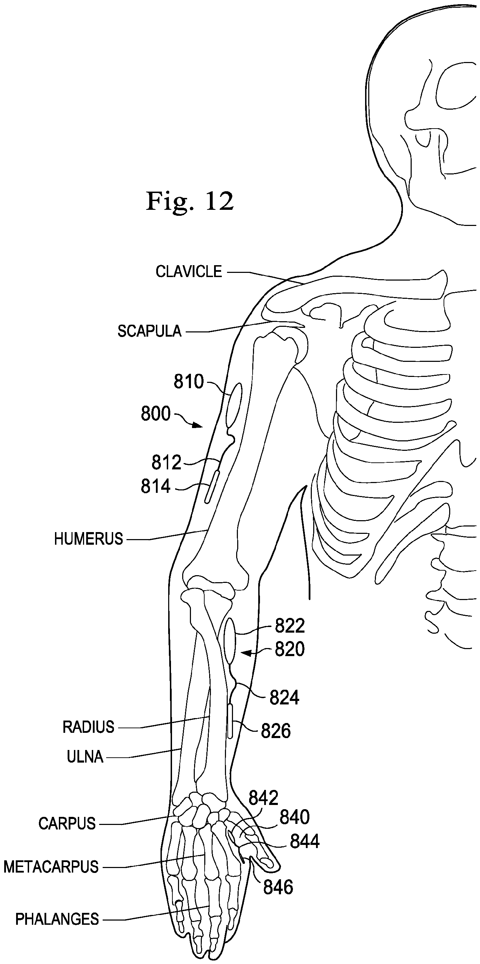

Although shown in Figs. 11a-11c for stimulation of the posterior tibial nerve, it is to be understood that the multi-stage neurostimulation system has application to a number of different peripheral nerve stimulation locations associated with Fig. 1 and the illustrations provided by the figures are merely exemplary. For example, Fig. 12 illustrates a stage one stimulation system 800 implanted along the humerous bone. The passive receiver 810, lead 812 and electrodes 814 all are positioned along the humerous bone without extending into or across the adjacent joints in the shoulder or elbow. Similarly, stage one stimulation system 820 is shown implanted along and within the length of the radius and ulna bones. Stimulation system 820 has the passive receiver 822, lead 824 and electrodes 826 implanted under the skin of the forearm but without any of the components extending into the adjacent joints of the elbow and the wrist. In still a further application, stimulation system 840 with passive receiver 842, lead 844 and electrodes 846 are implanted along a metacarpus bone in the hand in a configuration that is shorter than the length of the adjacent bone such that none of the components of the stimulation system extend across an adjacent joint in the wrist or the fingers. As previously described, and by way of example, the multi-stage neurostimulation system may be used in a patient's arms to stimulate one or more of the median, ulnar, radial, and brachial plexus nerves, as well as applied to a patient's legs to stimulate one or more of the tibial, saphenous, sciatic, and femoral nerves.

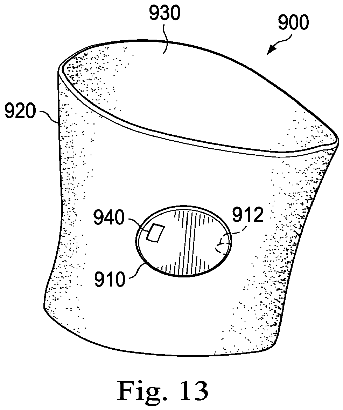

Referring now to Fig. 13, it is further contemplated that the system includes a device 900 to maintain the position of the EPG 910 in position on the body in relation to the passive receiver. As described above, the EPG 910 may include a socket 912 for receiving a connector of a cord to recharge the device and/or receive programming information. The EPG 910 also includes an accelerometer 940 to record patient limb movement. The device 900 includes flexible sleeve 920 that defines an interior passage 930 configured to go around an appendage to maintain the EPG adjacent a passive receiver involved in peripheral nerve stimulation. It is contemplated that the passage 930 will be configured and sized to substantially match the appendage of interest. Alternatively, the device 900 may be adjustable to fix the EPG in position. Still further, the EPG of Figs. 2 and 4a may be directly taped or otherwise fixed in position on the patient.

In another aspect, the above described systems may be used to treat temporary nociceptive limb pain. In certain orthopedic indications, such as hip, knee, ankle, shoulder, wrist and elbow problems, the patient is often advised by the attending physician to put off a surgical intervention until the pain is unbearable. In many instances, the patient is prescribed medication to mask the pain for as long as possible. However, systemic painkillers also impact the patient's quality of life making it difficult to work and drive, as well as causing other biologic side effects associated with long term use of pain killers. As an alternative, or to lessen the amount of painkillers need to relieve pain, in one non-limiting example, an electrode array, lead wire and passive receiver according to the present disclosure are implanted to relieve pain associated with the injured limb prior to a surgical intervention. As described above, the passive receiver is energized by an external pulse generator to provide neurostimulation to the related nerve(s). The use of the passive receiver system may continue for months or even years if the patient can still function in their daily lives. In the example of knee pain, the passive receiver system is implanted adjacent the femoral nerve between the hip and knee joints away from the injured site generating the pain. Neurostimulation continues prior to surgical intervention. In one form, the stimulation is generally continuous throughout the day. In another form, the stimulation may be used to relieve pain spikes caused by extensive use of the limb or during the evening to allow the patient to sleep comfortably. Once a decision is made to surgically address the cause of the pain, such as by a knee replacement, the system may be de-energized during the surgical procedure. However, the passive receiver may be reenergized shortly after surgery to mask at least part of the pain associated with the surgery. The neurostimulation system may continue to be used during the patient recovery period, which may last several months. Once the patient is no longer in pain, the passive receiver system may be removed from the patient, or the patient may elect to retain the system in the event they have future knee pain.

In a further example, a passive receiver system such as described above may be implanted in advance of limb amputation. Although the neurostimulator may be positioned to relieve pain prior to implantation, the primary objective will be to identify the nerves associated with phantom limb pain and begin stimulation of those nerves. In one form, it is anticipated that the pre-surgical stimulation would occur for approximately 4 weeks prior to amputation. Once it is determined that the patient is receiving pain blockage from the stimulation, the injured limb is then amputated. The stimulation system continues to operate to provide phantom limb pain relief to the patient after the limb has been amputated. In one form, it is anticipated that the neurostimulator would be operated for approximately 3 months after the amputation.

In a similar manner, the present neurostimulation system may also be temporarily implanted in association with reconstructive surgery. In such situations, it is common for the patient to undergo multiple surgical procedures over a long period of time. A neurostimulation system can be utilized to limit the amount of narcotics necessary to control the patient's pain. In addition to improving the patient's quality of life by limiting the amount of narcotics ingested, the system may also be more cost effective when compared to the total cost of drugs needed to achieve long term pain relief.

Thus, one method of using the limb peripheral nerve system is to implant a stimulation system to relieve chronic nociceptive pain prior to a surgical intervention to correct the injury causing the chronic pain. The method includes stimulating the nerve to relieve pain to delay surgical intervention, to prepare for amputation of a limb, or to provide relief during reconstructive surgeries. With the system implanted, a surgical intervention is performed to address the injured area causing pain. In one aspect, the passive receiver system is re-energized after surgery to assist in relieving post surgical pain.

In still a further aspect, a system and method for staged stimulation as described above can be applied to the vagal nerve, rather than being applied to the peripheral nerves, to provide a staged stimulation therapy. Similarly, a staged stimulation system and method as described above can be applied to other neural tissue such as the brain or spinal cord to provide a staged stimulation therapy.

The present invention, in various embodiments, includes providing devices and processes in the absence of items not depicted and/or described herein or in various embodiments hereof, including in the absence of such items as may have been used in previous devices or processes (e.g., for improving performance, achieving ease and/or reducing cost of implementation).

The foregoing discussion of the invention has been presented for purposes of illustration and description. The foregoing is not intended to limit the invention to the form or forms disclosed herein. In the foregoing Detailed Description for example, various features of the invention are grouped together in one or more embodiments for the purpose of streamlining the disclosure. This method of disclosure is not to be interpreted as reflecting an intention that the claimed invention requires more features than are expressly recited in each claim. Rather, as the following claims reflect, inventive aspects lie in less than all features of a single foregoing disclosed embodiment. Thus, the following claims are hereby incorporated into this Detailed Description, with each claim standing on its own as a separate preferred embodiment of the invention.

Moreover, though the description of the invention has included description of one or more embodiments and certain variations and modifications, other variations and modifications are within the scope of the invention (e.g., as may be within the skill and knowledge of those in the art, after understanding the present disclosure). It is intended to obtain rights which include alternative embodiments to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

In general, all features of the present specification may be combined with one another.

## Claims

1. A modular stimulation system for staged stimulation of nerves, comprising:
a plurality of stimulation electrodes;
a lead having a distal portion electrically coupled to said plurality of stimulation electrodes and an opposing proximal portion forming a first portion of a detachable coupling assembly;
a first stage implantable passive receiver stimulation module having a distal portion forming a second portion of a detachable coupling assembly complimentary to said first portion to electrically couple said passive receiver stimulation module to said lead, said passive receiver stimulation module configured to convert wireless energy to a pulse suitable to energize said plurality of stimulation electrodes; and
a second stage implantable pulse generator module having an internal power source and a controller to control delivery of electrical stimulation pulses, said implantable pulse generator module having a distal portion forming a third portion of a detachable coupling assembly complimentary with said first portion to electrically couple said implantable pulse generator to said lead,
wherein said passive receiver stimulation module and said implantable pulse generator module are configured to be sequentially coupled to said first portion of the detachable coupling.

2. The system according to claim 1, wherein said passive receiver module includes a receiver housing having a first displacement volume, and said implantable pulse generator module has a generator housing defining a second displacement volume, wherein said second volume is substantially the same as said first volume.

3. The system according to claim 1 or 2, wherein said plurality of leads includes at least two more leads than the passive receiver module can stimulate.

4. The system according to claim 2, wherein the first displacement volume is greater than 65% of the second displacement volume, or preferably greater than 75% of the second displacement volume, or even greater than 90% of the second displacement volume.

5. The system according to claim 2, wherein the first displacement volume is the same as the second displacement volume.

6. The system according to any one of the preceding claims, wherein the receiver housing includes a greater amount of unused space than the generator housing.

7. A modular neurostimulation system, comprising:
a plurality of stimulation electrodes;
a lead having a distal portion electrically coupled to said plurality of stimulation electrodes and an opposing proximal portion forming a first portion of a detachable coupling assembly;
an implantable passive receiver stimulator having a distal portion forming a second portion of a detachable coupling assembly complimentary to said first portion to electrically couple said passive receiver to said lead, said passive receiver configured to convert wireless energy to a pulse suitable to energize said plurality of stimulation electrodes; said passive receiver including an expansion connection; and
an implantable pulse generator having at least an internal power source or a controller to control delivery of electrical stimulation pulses, said implantable pulse generator having a portion forming a expansion coupling assembly complimentary with said expansion connection to electrically couple said implantable pulse generator to said passive receiver stimulator.

8. The system according to claim 7, wherein the passive receiver includes an implantable cover releasably covering said expansion connection.

9. The system according to claim 8, wherein the implantable pulse generator has a first displacement volume and said cover has a second displacement volume, said first displacement volume substantially matching said second displacement volume.

10. The system according to any one of claims 7-9, wherein said implantable pulse generator controls at least a portion of said passive receiver.

11. The system of according to any one of claims 7-10, wherein said passive receiver supplies at least one of power or data to the implantable pulse generator.

12. The system according to any one of claims 7-11, wherein the implantable pulse generator is configured to operate utilizing said internal power source unless external power is supplied to said passive receiver.

13. The system according to any one of claims 7-12, wherein said implantable pulse generator is configured to operate utilizing said internal power source or power delivered wirelessly through said passive receiver.

14. The system according to claim 13, wherein said implantable pulse generator is configured to charge said internal power source with power delivered wirelessly through said passive receiver.
